Europäisches Patentamt

**European Patent Office**

**Office européen des brevets**

(11) Publication number: **0 293 502 B1**

# EUROPEAN PATENT SPECIFICATION

(45) Date of publication of patent specification: **30.09.92**  (51) Int. Cl.⁵: **G01N 33/20**

(21) Application number: **87107977.8**

(22) Date of filing: **02.06.87**

## (54) Method for analysis of molten metal.

(43) Date of publication of application:
**07.12.88 Bulletin 88/49**

(45) Publication of the grant of the patent:
**30.09.92 Bulletin 92/40**

(84) Designated Contracting States:
**BE CH DE ES FR GB IT LI NL SE**

(56) References cited:
**EP-A- 0 000 926**
**CA-A- 1 179 017**
**FR-A- 2 579 326**
**GB-A- 1 116 052**
**US-A- 3 668 934**

(73) Proprietor: **ALUMINUM COMPANY OF AMER-
ICA**
**1501 Alcoa Building Mellon Square
Pittsburgh, PA 15219(US)**

(72) Inventor: **Genna, John Lawrence**
**139 Wiltshire Circle
Monroeville, PA(US)**

(74) Representative: **Baillie, Iain Cameron et al
c/o Ladas & Parry, Altheimer Eck 2
W-8000 München 2(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a method for analysis of molten metal. More particularly, this invention relates to a method in which a solid sample is removed from a source of molten metal; dissolved; and the dissolved sample then analyzed.

In the production of metal alloys such as, for example, an aluminum base alloy, it is desirable to determine the alloy content while the alloy is still in molten form. This, in turn, permits the addition of further alloying materials, or more base metal if the alloying metals are more concentrated than desired, while the alloy mixture is still molten.

Various methods of analyzing such molten metals are possible. In its simplest form, such analysis could comprise taking a sample from the melt and sending it out for spectral analysis. This, of course, would involve an unacceptable time lag. On the other hand, however, direct spectral analysis of the molten metal would create problems if sensitive spectrometer equipment was located in immediate proximity to a furnace containing molten metal.

Various alternative methods of analysis have been previously proposed. For example, Bojic U.S. Patent 3,659,944 describes a system wherein a stream of molten metal is drawn into a spark chamber where the molten metal comes in contact with one electrode. The light or radiation generated by a spark between the molten metal and a second electrode is directed into a spectrometer to provide direct analysis of the molten metal. However, such a method involves the transport of molten metal from a furnace to the spark chamber and further results in the need to transmit the emitted spectra to the spectrometer if the spark chamber is located near the molten metal.

Virgolet U.S. Patent 3,669,546 illustrates a system for analysis of molten metal wherein one electrode is placed directly into the molten metal bath and an electric arc is generated between the surface of the bath and another electrode placed adjacent the surface. The light emitted from this electric arc is then transmitted by a series of mirrors to a spectrograph where the light is analyzed to determine the content of the molten metal. Similar systems are disclosed in Bojic et al U.S. Patents 3,645,628 and 3,672,774 wherein light is produced by generating sparks between an electrode and the surface of a crucible filled with molten metal and in contact with a second electrode. The light thus produced is directed to a spectrometer for analysis. Such systems, however, require the transmission of the emitted light to a spectral analysis apparatus spaced some distance from the furnace and thus some distance from the point of generation of the light.

British Patent Specification 1,116,052 shows a mechanism for analyzing molten material by passing a gas under pressure into the molten metal to produce metal particles which are then transported out of the bath to a spectrograph for analysis by feeding the particles or dust into a plasma jet. Production of metal particles from molten metal is also shown by Maringer U.S. Patent 4,154,284 who teaches the production of metal particles such as metal flake by dipping a portion of a rotating wheel into a pool of molten metal. The wheel is provided with sawtoothlike serrations which pick up the molten metal as the wheel passes through the molten metal pool. As the wheel emerges from the molten metal, centrifugal force and/or contact with gases cause the now solidified metal to break off as flakes from the rotating wheel. The serrated surface of the wheel may also be cleared of any adhering metal from the molten metal pool by contacting the serrations with a brush.

Canadian Patent 1179017 is concerned with the dissolving of solid samples for analysis purposes. It does not, however, either disclose or suggest an integrated system permitting analysis at a remote location.

Kenney International Application PCT/US84/01148, however, points out that problems such as interruption of particle flow due to clogging can occur in attempting to transport such metal powder. Instead, Kenney proposes a system for analysis of molten metal wherein an atomization die is used in connection with pressurized inert gas to form an aerosol or dispersion of solidified metal particles in the gas. This aerosol or dispersion is then delivered to an inductively coupled plasma torch which causes the particles to emit spectra characteristic of their constituent elements which may then be analyzed with a spectrometer.

It would, however, be preferable to provide a system for on-line analysis of the contents of a molten metal bath at a position remote from the bath which was not dependent on the transporting of either emitted spectra or powders and/or aerosols to the remove spectral analysis position.

It is therefore an objective of this invention to provide an improved method for the analysis of a molten metal source.

It is a further objective of this invention to provide an improved method for the analysis of a molten metal source wherein a sample of solid metal particles from the molten metal source is removed from the source, dissolved in a solvent, and then transported to a spectrometer for analysis.

According to the present invention there is provided a method for analyzing molten metal in an integrated system characterized by the steps of

    (a) removing sample metal as a solid from a source of molten metal by immersing serrations of a rotating disc into said source of molten metal;

(b) transporting the sample metal by suction from the disc to a dissolution zone;

(c) dissolving a measured quantity of the sample metal in a solvent in said dissolution zone to form a dissolved sample;

(d) passing the dissolved sample from the dissolution zone to an emission spectrometer;

(e) vaporizing said dissolved sample in the spectrometer to form a vaporized sample;

(f) producing an emission spectra from said vaporized sample; and

(g) comparing the emission spectra of the vaporized sample to an emission spectra of a known amount of a known alloy of the same metal as the sample metal to determine the composition of the sample metal.

According to a further aspect of the invention the method of the precedign paragraph is for analyzing the content of a molten aluminum alloy source and is characterized by the steps of

(a) removing a sample of aluminum alloy from said source in a sampling zone by:

(1) contacting said source of molten aluminum alloy with a spinning disc having serrations located along a periphery thereof by partially immersing said disc into said molten aluminum alloy source; and

(2) collecting solidified aluminum alloy from said disc by dislodging solid aluminum alloy from said serrations as said serrations emerge from said molten aluminum alloy source to form said sample of aluminum alloy;

(b) transporting said aluminum alloy sample by suction to a dissolution zone from the disc in said sampling zone;

(c) dissolving a known amount of said aluminum alloy sample in a solvent in said dissolution zone to form a dissolved aluminum alloy sample;

(d) directing the dissolved aluminum alloy sample to an emission spectrometer;

(e) analyzing the dissolved aluminum alloy sample in said emission spectrometer by:

(1) vaporizing said dissolved aluminum alloy sample to form a vaporized sample;

(2) producing an emission spectra from said vaporized sample; and

(3) comparing the emission spectra of said vaporized sample to an emission spectra of a known amount of aluminum alloy of known alloy content.

The invention will be described with reference to the accompanying drawings in which:

Figure 1 is a partial cross-section view of an apparatus for use in the method of the invention which comprises a sampling zone that includes a sampling wheel,

Figure 2 is a schematic representation of a further apparatus for use in the method which comprises a sample processing chamber, which chamber is a dissolution zone and compartment,

Figure 3 is a diagrammatic presentation of a system which may be used in performing the method of the invention;

As hereinbefore described the preferred method of the invention provides for the collection of solid metal particles from a source of molten metal, the fluid transmission of these particles to a dissolution zone, the dissolving of the solid metal particles in a solvent, passing the solution to a spectral analysis zone, vaporization of the solution, and analysis of the emitted spectra to determine the contents of the molten metal source.

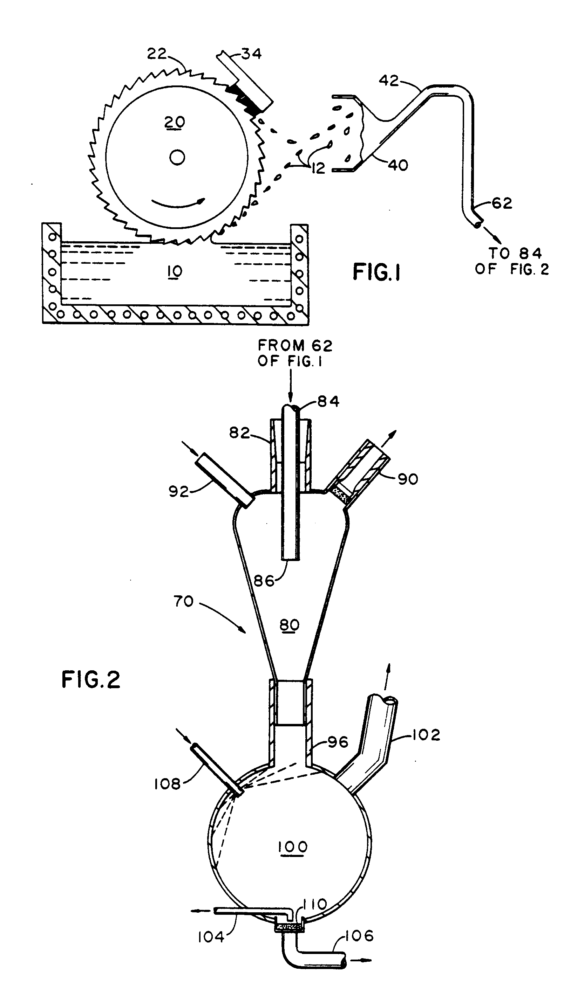

In a preferred embodiment, as illustrated in Figure 1, solid metal particles 12 are collected form the molten metal source 10 by partially immersing a spinning wheel 20 into the source. Wheel 20 may, for example, comprise an 11-inch diameter copper wheel, preferably with serrations or sawtooth edges 22. Wheel 20 is lowered into molten metal source 10 to a depth of, preferably, no greater than 0.5 millimeter. At the same time, wheel 20 is rotated at a speed of from about 200 rpm. As wheel 20 rotates, molten metal solidifies in the serrations 22, contracts, and is ejected from wheel 20 by centrifugal forces to be collected in hood means 40, as will be described below. A brush 34 located in the manner shown in Figure 1 may be used to assist in removal of metal particles from serrations 22 if desired.

Preferably, serrations 22 comprise sawtooth-shaped edges each having a leading edge of about 0.078 inch and a length of about 5.6 millimeter (0.22 inch) with the hypotenuse of the sawtooth forming a 20° angle with the length to thereby provide about 150 to 160 serrations on an 0.28 meter (11-inch) diameter wheel. With these dimensions, it is possible to produce metal flakes having a range of particle sizes from approximately 0.12 to 0.4 cm in length, 0.04 to 0.15 cm in width, and 0.016 to 0.020 cm thick with an approximate weight of between 0.00026 to 0.004 grams per flake.

A fairly reproducible or known amount of metal particle or flake sample can thus be collected by lowering wheel 20 into molten metal source 10 for a measured increment of time and at a known speed of rotation and then raising wheel 20 out of the pool of molten metal. Alternatively, the flakes or particles can be weighed in a weight station with an increment of sample transported to the dissolution zone only when a certain weight of sample is reached.

Solid metal flakes or particles 12, as they dislodge from serrations 22 in wheel 20, are collected by hood means 40 which is positioned in the path of travel of the particles. Hood means 40 is connected, via tube or pipe 42, to a tube or pipe 62 that delivers the particles to a processing chamber 70 (Figure 2). A vacuum is produced in 70 and in a compartment 80 of 70 by means of a blower means (not shown) attached to an exit port 90 (Figure 2) of chamber 70. This vacuum causes metal particles to be transported from hood 40 through pipes 42 and 62 into the sample processing chamber. Tube 62 can be of substantial length to allow processing of flakes 12 at a location remote from that of their origin.

Sample processing chamber 70 includes a first compartment 80 having an inlet opening 82 connected to tube 62, which opening includes an inlet tube 84 that protrudes into compartment 80. 84 Terminates in an opening 86 which faces downwardly in compartment 80. The metal particles entering compartment 80 thus lose momentum and fall through compartment 80 into a dissolution chamber 100 via port 96 located below 80.

The dissolution chamber 100 is provided with a solvent inlet 108 through which a measured amount of solvent is admitted into the chamber to dissolve the metal particles entering through ports 82 and 96. The solvent is dispensed in chamber 100 in such a way as to provide a washdown of the sides of dissolution vessel 100.

A lower portion of dissolution compartment 100 has a sample solution outlet 104 and a drain 106. Drain 106 contains a porous frit of a material 110 that does not react with the solvent. Frit 110 prevents solid materials from leaving compartment 100. A point tube 102 is shown connected to an upper side portion of compartment 100 to vent out gases that may be produced as the solid particles are dissolved. This venting is promoted by utilizing a flow of air to and through a nipple 92 during dissolution, nipple 92 being connected to an upper portion of compartment 80.

The solvent used to dissolve the metal particles preferably is a mineral acid such as a 50% HCl solution. Any other solvent capable of rapidly dissolving the metal sample may, however, be used. The term "rapidly" means the use of a solvent capable of dissolving the sample in about 30 seconds or less. For example, when a sample of aluminum flakes weighing approximately 0.1 gram is used, 10 milliliters of 50% HCl will dissolve the sample in about 10 seconds.

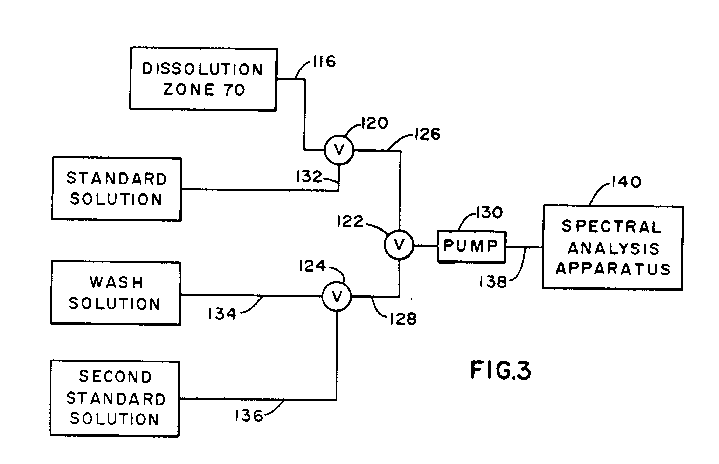

After the solid metal sample is dissolved, the sample solution is pumped out of dissolution compartment 100 through outlet 104 and into spectral analysis apparatus 140 (Figure 3) in a manner described below. It should, however, be noted here that as soon as the dissolved sample leaves dissolution compartment 100 another sample may begin to be collected by again dipping wheel 20 into the molten metal to permit the collection and transport to dissolution zone or chamber 70 of a new sample of solidified metal for eventual analysis. In this manner, an on-line or semi-continuous measurement of the contents of a molten metal source may be maintained.

Spectral analysis apparatus 140 may comprise any conventional spectrometer capable of vaporizing a liquid sample. Such spectral analysis equipment, conventionally known as an Inductively Coupled Emission Spectrometer, is commercially available.

Referring now to Figure 3, the components of which are only schematically represented, a tube 116 leads from outlet 104 to a first valve 120. Valve 120 is a three-way valve which has a second inlet 132 connecting valve 120 with a standard solution which may be used to calibrate spectral analysis apparatus 140. A tube 126 connects the outlet of valve 120 with one inlet of a second three-way valve 122. A second inlet of valve 122 is connected via tube 128 to the outlet of a third three-way valve 124. Valve 124 has an inlet 134 connected to a source of wash solution which may be pumped into spectral analysis apparatus 140 to clean the apparatus between runs. A second inlet 136 to valve 124 connects to an optional second standardized solution used to calibrate apparatus 140. Valves 120, 122, and 124 preferably are solenoid-operated valves which may be controlled by a central control unit (not shown) and which may also be used to control the remainder of the sampling apparatus, including wheel 20 and the input of solvent into dissolution zone 70.

Connected to the outlet of valve 122 via tube 138 is a pump 130 which will pump into spectral analysis apparatus 140 whichever solution is set to pass through valves 120, 122, and 124 depending upon the settings of the valves.

The sample solution then, passes through outlet 104 and tube 116 from dissolution zone 70 into valve 120 and then via tube 126 into valve 122 from whence it passes through pump 130 into spectral analysis apparatus 140. The sample solution then is vaporized in spectral analysis apparatus 140 and the contents of the molten metal source are determined.

In summary, the method of the invention provides for the analysis of a molten metal source by removing a solid sample from the molten metal source, transporting the sample to a dissolution zone in a fluidized medium, and then passing to a spectral analysis apparatus a solution containing the sample. In this manner known amounts of sample may be analyzed to determine the amount of alloying materials in the molten metal in a rapid yet accurate manner. The various zones can be controlled by a central control unit such as a process control computer and the zones may sequentially process different samples to thereby accelerate the overall monitoring of the content of the molten metal source.

## Claims

1. A method for analyzing molten metal in an integrated system characterized by the steps of
   (a) removing sample metal as a solid from a source of molten metal by immersing serrations of a rotating disc into said source of molten metal;
   (b) transporting the sample metal by suction from the disc to a dissolution zone;
   (c) dissolving a measured quantity of the sample metal in a solvent in said dissolution zone to form a dissolved sample;
   (d) passing the dissolved sample from the dissolution zone to an emission spectrometer;
   (e) vaporizing said dissolved sample in the spectrometer to form a vaporized sample;
   (f) producing an emission spectra from said vaporized sample; and
   (g) comparing the emission spectra of the vaporized sample to an emission spectra of a known amount of a known alloy of the same metal as the sample metal to determine the composition of the sample metal.

2. A method according to claim 1, characterized in that the sample metal is in the form of solidified metal particles or flakes which are formed in the serrations of the disc when the serrations are removed from the source of molten metal.

3. A method according to claim 1, characterized in that the step of removing the sample metal as a solid includes removing said sample metal from said serrations on said rotating disc by contacting said disc with means for dislodging said sample metal from said serrations on said disc.

4. A method according to claim 3, characterized in that the dislodging means comprises a brush or an air jet.

5. A method according to claim 1, characterized in that the sample metal removed from the source of molten metal is in the form of solid particles or flakes.

6. A method according to claim 1, for analyzing the content of a molten aluminum alloy source, characterized by the steps of
   (a) removing a sample of aluminum alloy from said source in a sampling zone by:
      (1) contacting said source of molten aluminum alloy with a spinning disc having serrations located along a periphery thereof by partially immersing said disc into said molten aluminum alloy source; and
      (2) collecting solidified aluminum alloy from said disc by dislodging solid aluminum alloy from said serrations as said serrations emerge from said molten aluminum alloy source to form said sample of aluminum alloy;
   (b) transporting said aluminum alloy sample by suction to a dissolution zone from the disc in said sampling zone;
   (c) dissolving a known amount of said aluminum alloy sample in a solvent in said dissolution zone to form a dissolved aluminum alloy sample;
   (d) directing the dissolved aluminum alloy sample to an emission spectrometer;
   (e) analyzing the dissolved aluminum alloy sample in said emission spectrometer by:
      (1) vaporizing said dissolved aluminum alloy sample to form a vaporized sample;
      (2) producing an emission spectra from said vaporized sample; and
      (3) comparing the emission spectra of said vaporized sample to an emission spectra of a known amount of aluminum alloy of known alloy content.

## Patentansprüche

1. Verfahren zur Analyse von schmelzflüssigem Metall in einem integrierten System, gekennzeichnet durch die Schritte:
   (a) Entnehmen einer Metallprobe in fester Form von einer Quelle schmelzflüssigen Metalls, indem Kerben einer rotierenden Scheibe in besagte Quelle schmelzflüssigen Metalls eintauchen;
   (b) Transportieren der Metallprobe von der Scheibe zu einer Auflösungszone durch Ansaugen;
   (c) Auflösen einer abgemessenen Menge der Metallprobe in einem Lösungsmittel in besagter Auflösungszone, um eine aufgelöste Probe zu bilden;
   (d) Überleiten der aufgelösten Probe von der Auflösungszone zu einem Emissionsspektrometer;
   (e) Verdampfen besagter aufgelöster Probe in dem Spektrometer, um eine verdampfte Probe zu bilden;

(f) Erzeugen eines Emissionsspektrums besagter verdampfter Probe;

(g) Vergleichen der Emissionsspektren der verdampften Probe mit einem Emissionsspektrum einer bekannten Menge einer bekannten Legierung des gleichen Metalls, wie das Metall der Probe, um die Zusammensetzung des Metalls der Probe zu bestimmen.

2. Verfahren nach Anspruch 1, dadurch gekennzeichnet, daß das Metall der Probe in Form verfestigter Metallteilchen oder Plättchen vorliegt, die in den Kerben der Scheibe gebildet werden, wenn die Kerben aus der Quelle des schmelzflüssigen Metalls entfernt werden.

3. Verfahren nach Anspruch 1, dadurch gekennzeichnet, daß der Schritt zur Entfernung des Probenmetalls als ein Feststoff die Entfernung der besagten Metallprobe von den besagten Kerben auf der besagten rotierenden Scheibe umfaßt, indem die besagte Scheibe mit einer Einrichtung zur Entfernung des besagten Probenmetalls von besagten Kerben auf besagter Scheibe versehen ist.

4. Verfahren nach Anspruch 3, dadurch gekennzeichnet, daß die Einrichtung zur Entfernung eine(n) Bürste oder Luftstrahl umfaßt.

5. Verfahren nach Anspruch 1, dadurch gekennzeichnet, daß das Metall der Probe, welches von der Quelle des schmelzflüssigen Metalls entfernt wird, in Form fester Teilchen oder Plättchen vorliegt.

6. Verfahren nach Anspruch 1 zur Analyse des Gehalts einer schmelzflüssigen Aluminiumlegierungsquelle, gekennzeichnet durch die Schritte:

(a) Entnehmen einer Probe einer Aluminiumlegierung von besagter Quelle in einer Probenahmezone durch:

(1) Kontaktieren besagter Quelle der schmelzflüssigen Aluminiumlegierung mit einer drehenden Scheibe, die an ihrem Umfang Kerben hat, durch teilweises Eintauchen besagter Scheibe in die schmelzflüssige Aluminiumlegierungsquelle; und

(2) Aufnehmen der verfestigten Aluminiumlegierung von der besagten Scheibe durch Entfernen der festen Aluminiumlegierung von besagten Kerben, wenn besagte Kerben aus der besagten schmelzflüssigen Aluminiumlegierungsquelle auftauchen, um besagte Probe der Aluminiumlegierung zu bilden;

(b) Transportieren der besagten Aluminiumlegierungsprobe durch Ansaugen zu einer Auflösungszone von der Scheibe in besagte Probenahmezone;

(c) Auflösen einer bekannten Menge besagter Aluminiumlegierungsprobe in einem Lösungsmittel in besagter Auflösungszone, um eine aufgelöste Aluminiumlegierungsprobe zu bilden;

(d) Überleiten der aufgelösten Aluminiumlegierungsprobe zu einem Emissionsspektrometer;

(e) Analysieren der aufgelösten Aluminiumlegierungsprobe in besagtem Emissionsspektrometer durch:

(1) Verdampfen besagter aufgelöster Aluminiumlegierungsprobe, um eine verdampfte Probe zu bilden;

(2) Erzeugen eines Emissionsspektrums von besagter verdampfter Probe; und

(3) Vergleichen der Emissionsspektren besagter verdampfter Probe mit einem Emissionsspektrum einer bekannten Menge Aluminiumlegierung mit bekannter Legierungszusammensetzung.

**Revendications**

1. Procédé d'analyse de métal fondu dans un système intégré, caractérisé par les étapes de

(a) prélèvement d'un métal-échantillon sous la forme d'un solide à partir d'une source de métal fondu par immersion des dents d'un disque tournant dans ladite source de métal fondu ;

(b) transport du métal-échantillon, par aspiration, du disque à une zone de dissolution ;

(c) dissolution d'une quantité mesurée du métal-échantillon dans un solvant dans ladite zone de dissolution pour former un échantillon dissous ;

(d) passage de l'échantillon dissous de la zone de dilution à un spectromètre d'émission ;

(e) vaporisation dudit échantillon dissous dans le spectromètre pour former un échantillon vaporisé ;

(f) production d'un spectre d'émission dudit échantillon vaporisé et

(g) comparaison du spectre d'émission de l'échantillon vaporisé à un spectre d'émission d'une quantité connue d'un alliage connu du même métal que le métal-échantillon, pour déterminer la composition du métal-échantillon.

2. Procédé selon la revendication 1, caractérisé en ce que le métal-échantillon se présente sous la forme de particules ou flocons de métal solidifié qui sont formés dans les dents du disque lorsque les dents sont retirées de la source de métal fondu.

3. Procédé selon la revendication 1, caractérisé en ce que l'étape de prélèvement du métal-échantillon sous la forme d'un solide comprend l'enlèvement dudit métal-échantillon desdites dents sur ledit disque tournant par mise en contact dudit disque avec un moyen de délogement dudit métal-échantillon desdites dents sur ledit disque.

4. Procédé selon la revendication 3, caractérisé en ce que le moyen de délogement comprend une brosse ou un jet d'air.

5. Procédé selon la revendication 1, caractérisé en ce que le métal-échantillon prélevé sur la source de métal fondu se présente sous la forme de particules ou flocons solides.

6. Procédé selon la revendication 1 d'analyse de la teneur d'une source d'alliage d'aluminium fondu, caractérisé par les étapes de
   (a) prélèvement d'un échantillon d'alliage d'aluminium dans ladite source dans une zone d'échantillonnage par :
      (1) mise en contact de ladite source d'alliage d'aluminium fondu avec un disque en giration rapide présentant des dents situées le long de sa périphérie, par immersion partielle dudit disque dans ladite source d'alliage d'aluminium fondu et
      (2) recueil de l'alliage d'aluminium solidifié sur ledit disque par délogement de l'alliage d'aluminium solide desdites dents lorsque lesdites dents émergent de ladite source d'alliage d'aluminium fondu pour former ledit échantillon d'alliage d'aluminium ;
   (b) transport dudit échantillon d'alliage d'aluminium, par aspiration, du disque dans ladite zone d'échantillonnage à une zone de dissolution ;
   (c) dissolution d'une quantité connue dudit échantillon d'alliage d'aluminium dans un solvant dans ladite zone de dissolution pour former un échantillon d'alliage d'aluminium dissous ;
   (d) amenée de l'échantillon d'alliage d'aluminium dissous à un spectromètre d'émission ;

(e) analyse de l'échantillon d'alliage d'aluminium dissous dans ledit spectromètre d'émission par :
   (1) vaporisation dudit échantillon d'alliage d'aluminium dissous pour former un échantillon vaporisé ;
   (2) production d'un spectre d'émission dudit échantillon vaporisé et
   (3) comparaison du spectre d'émission dudit échantillon vaporisé avec un spectre d'émission d'une quantité connue d'alliage d'aluminium ayant une teneur connue en alliage.

FIG.1

FIG.2

FROM 62
OF FIG.1

TO 84
OF FIG. 2

FIG.3